# EUROPEAN PATENT SPECIFICATION

(11) **EP 1 715 801 B1**
(45) Date of publication and mention of the grant of the patent: **30.05.2012**
(21) Application number: 05715347.0
(22) Date of filing: 14.02.2005
(51) Int. Cl.: A61B 19/00, A61F 2/46

(54) **INSTRUMENT FOR THE GEOMETRICAL EVALUATION OF INTERNAL LESIONS**
INSTRUMENT ZUR GEOMETRISCHEN BEURTEILUNG VON GEWEBE-LÄSIONEN
INSTRUMENT DESTINE A L'EVALUATION GEOMETRIQUE DE LESIONS INTERNES

(30) Priority: 17.02.2004 IT MI20040260
(43) Date of publication of application: 02.11.2006
(73) Proprietor: Politecnico Di Milano, 20133 Milano (IT)
(72) Inventor: RAIMONDI, Manuela, Teresa, I-20149 Milano (IT)
(74) Representative: Colombo, Stefano Paolo
(86) International application number: PCT/EP2005/001538
(87) International publication number: WO 2005/077292

(56) References cited:
- EP-A- 0 824 893
- US-A- 5 950 320
- US-A1- 2004 092 949
- US-A1- 2004 267 164

## Description

The present invention concerns the biomedical sector and so-called mini-invasive surgery. In particular it relates to an instrument for performing evaluations and mapping lesions affecting biological tissues or internal organs. In one particular application, the present invention relates to the mapping of articular cartilage lesions.

For some years surgical operations which are minimally invasive, referred to in short as "mini-invasive" operations, have been carried out. For example, an arthroscopic operation envisages the introduction, into the articulation, of an optical system which typically consists of an optical fibre connected to an image acquisition system (arthroscopic monitor) and a light source for illuminating the operating site, and a system for entry of a washing fluid (usually a water-based physiological solution). On the other side of the articulation a special arthroscopy guide is introduced, said guide being equipped with an outlet for drawing off the washing fluid and keeping the surgical field clean. The surgeon introduces surgical instruments such as drills, milling cutters or the like inside the guide.

Substantially non-invasive diagnostic systems, such as computerized axial tomography (CAT) or magnetic resonance, which allow high-definition radiological examination of organs inside a human or animal body, are also known.

However, the cartilaginous tissues, which basically consist of chondrocytes and fibres which are collagenous and elastic but substantially not vascularized, are poorly visible when using the abovementioned diagnostic systems. The problem of not being able to see the cartilaginous tissue when using CAT or magnetic resonance is particularly critical in the case of cartilaginous lesions of the chondral or osteochondral type. These lesions are usually of a traumatic origin and in current clinical practice they are diagnosed mainly by means of arthroscopic investigation since the non-invasive diagnostic methods which are currently available do not allow a reliable diagnosis. During arthroscopic investigation, a surgeon has the possibility of observing directly the lesion on an arthroscopic monitor, but does not have any system which allows him/her to make a quantitative evaluation of the form and size thereof.

Similar situations in which it is practically impossible to evaluate in a quantitative and reliable manner the size and form of an object in a human or animal body may be, for example, those relating to ulcers or focal lesions of tissues or internal organs. The present invention will be described principally with reference to lesions of cartilaginous tissues, in particular lesions of cartilaginous tissues of the knee. However, it is not to be understood in any way as being limiting to this area.

At present, chondral lesions are described on the clinical record of a patient in a qualitative manner, using only a classification diagram based on a subjective visual assessment of the depth of the lesion.

The impossibility of assessing the extent and the form of chondral lesions in a non-subjective manner gives rise to a series of problems in the clinical, scientific and medical/legal sectors.

In the clinical sector, novel tissue repair therapies using (primary and stem) cells have been proposed and have already reached the stage of clinical experimentation on man. In treatment of cartilaginous lesions of the knee, the therapy envisages filling the lesion with a solid construction containing the cells. A problem relating to the conversion of these techniques such that they are arthroscopic rather than arthrotomic consists in the need to acquire the form and the size of the lesion so that it may then be filled with a made-to-measure patch.

In the scientific field it is currently not possible to convert, from a qualitative to a quantitative nature, the evaluation of the clinical result in the present therapies for treatment of chondral lesions. This in turn results in the drawback of not being able to optimize in a more reliable manner the diagnosis as to the choice of the most effective treatment for different types of lesions.

Some types of chondral lesions cannot, however, be treated using conventional therapies which are currently available in clinical practice. Typically very extensive lesions which are situated in zones subject to a high articular load cannot be treated. A surgeon may diagnose them during an arthroscopic examination, but is unable to treat them directly at the time. This means that a few months after the examination the same symptoms which led to the surgical operation reappear in the patient. The problem therefore consists in the fact that the surgeon is unable to document on the clinical record the findings of the arthroscopy so that he/she is protected against subsequent claims or legal action on the part of the patient.

US 6,592,588 and US 6,591,581 refer to a surgical technique for the treatment of small-size chondral lesions. The technique in question comprises the step of removing from the patient healthy cartilage disks, in a zone of the articulation which is not subject to load, and transferring them into the lesion to be treated. Both patents describe arthroscopic measuring instruments which allow an assessment of the depth of the lesion at a given point and definition of the diameter of supposed circular shaped lesions. The two patents do not provide, however, any information as to mapping of the lesion and an evaluation as to its real size and form.

US 6,610,067 relates to an implant device for treating chondral lesions of the knee and the associated set of surgical implantation instruments. It describes a series of instruments for defining a direction perpendicular to the articular surface in the zone for application of a prosthetic device, which fills the lesion, and an instrument of the compass type, which allows an estimation as to the radius of a lesion, once prepared in a circular form so as to be able to receive the prosthesis. Essentially, these instruments have the function of implanting the prosthesis correctly. The patent also describes an electronic apparatus for performing digital mapping of the articular surface in the area of the lesion. The instrument is designed to allow, via manual sampling of random trajectories with an arthroscopic contact probe, acquisition of the coordinates of points on the surface which are then reprocessed for subsequent reconstruction of the three-dimensional geometry by means of interpolation.

EP 0 824 893 discloses an apparatus for osteochondral autografit transplantation.

The main object of the present invention is therefore that of solving the drawbacks and problems mentioned above and providing a method and a device for performing evaluations and mapping lesions of biological tissues or internal organs.

These and other objects of the invention are achieved by an instrument according to the independent Claim 1. Further advantageous features of the invention are contained in the dependent claims. All the claims are understood as forming an integral part of the present description.

The invention allows mapping of a lesion using an arthroscopic surgical instrument able to arrange on top of the surface affected by the lesion a geometrical sampling system such as, for example, a screen. This sampling system is clearly visible on the arthroscopic monitor and therefore allows recording and archiving, for subsequent evaluation, of a digital image which shows the sampling system superimposed on the lesion; and tracing, also manually, of a precise map of the lesion.

According to a first aspect, the present invention relates to a surgical instrument for geometrical evaluation of an object inside a body of a human being or animal, the instrument comprising a handle, a reference device and means for bringing said reference device into the vicinity of said object, said instrument co-operating with an image acquisition device for acquiring images of said reference device in the vicinity of said object.

In one particular application thereof, said object is a lesion of an internal tissue, typically a cartilage tissue of an articulation or the like.

According to one embodiment of the present invention, said reference device is a screen or the like.

Preferably, said reference device is a colour substantially contrasting with the object to be evaluated geometrically. For example, if the instrument according to the invention is applied to the geometrical evaluation of cartilage lesions, the reference device has a dark grey or black colour.

According to one embodiment of the invention, the surgical instrument comprises a guide barrel and a shank sliding inside the barrel, the barrel being able to be inserted inside the body, possibly using an arthroscopic guide or the like and having a proximal end in the vicinity of the handle and an opposite distal end which is open.

Conveniently the shank has a head-piece with support arms for supporting said reference device.

The arms are pivotably mounted and are elastically movable between a first position where the reference device is retracted and a second position where the reference device is unfolded in the vicinity of the object to be evaluated.

The shank is moved from the position were the reference device is retracted inside the barrel into the position where the reference device is unfolded in the vicinity of the object to be evaluated by means of a trigger situated at the proximal end.

A recall system for recalling the reference device from the unfolded position into the retracted position is preferably provided.

In one embodiment, the recall system comprises a wire connected to the reference device and to the handle.

Conveniently, the wire is slidable inside an axial cavity of the shank.

The handle is preferably of the "scissors type".

In order to ensure that the device is sterile, light and low-cost, the surgical instrument is at least partially made with polymer material and is of the disposable type.

The invention will become fully clear from the detailed description which follows, provided purely by way of a non-limiting example, to be read with reference to the accompanying plates of drawings, in which:
- Fig. 1 shows in schematic form the device according to the invention, partially sectioned, during use for evaluating the geometry of a cartilage lesion;
- Fig. 2 shows a cross-section performed along the line 1-1 of Fig. 1;
- Fig. 3 shows in schematic form the device according to the invention, partially sectioned, during insertion or extraction into/from a body;
- Fig. 4 is a cross-section performed along the line 3-3 in Fig. 3; and
- Fig. 5 shows in schematic form an application of the method according to the invention for suitably shaping a tissue produced in a laboratory.

As mentioned above, an application of the present invention with reference to the evaluation of the dimensions and form of cartilage tissue lesions, in particular those affecting the knee, is described. However, it is not to be regarded in any way as being limited to this area.

The invention comprises the step of introducing, in a closed-cover operating environment, a sampling device or else said reference device with known dimensions. Conveniently, a screen with meshes of a predetermined size is used, for example a screen with square shaped meshes having a side with dimensions of about 0.5 mm - 1.5 mm, typically about 1.0 mm. In the case where the present invention is used for mapping cartilage lesions, the screen has a colour contrasting with the whitish light colour of the cartilage. Typically it has a dark grey or black colour.

The screen is brought into the vicinity of the lesion by means of a surgical instrument. Conveniently, the screen is brought into the operating site by means of an arthroscopic guide, the same guide which is used to introduce surgical instruments and which is already usually inserted inside the body by a surgeon. An embodiment of the surgical instrument according to the present invention has been illustrated in the various figures accompanying the present description. For the purposes of the present patent application, the term "proximal" will be understood as meaning "close to the surgeon" (or in any case close to the person gripping or operating the surgical instrument) and the term "distal" will be understood as meaning "far from the surgeon". When the device is not in use, the term "proximal" will in any case refer to the part closest to the handle.

The instrument 1 comprises a handle 2, a barrel 3 and a shank 4 sliding inside the barrel. The distal end of the barrel 3 is able to be inserted inside the body, if necessary through the arthroscopic guide 6, and is substantially open. Preferably, the arthroscopic guide 6, the barrel 3 and the shank 4 are coaxial. A space is provided between the internal surface of the arthroscopic guide 6 and the barrel 3, so as to allow a washing fluid (typically a water-based physiological solution) to flow towards an outlet 61.

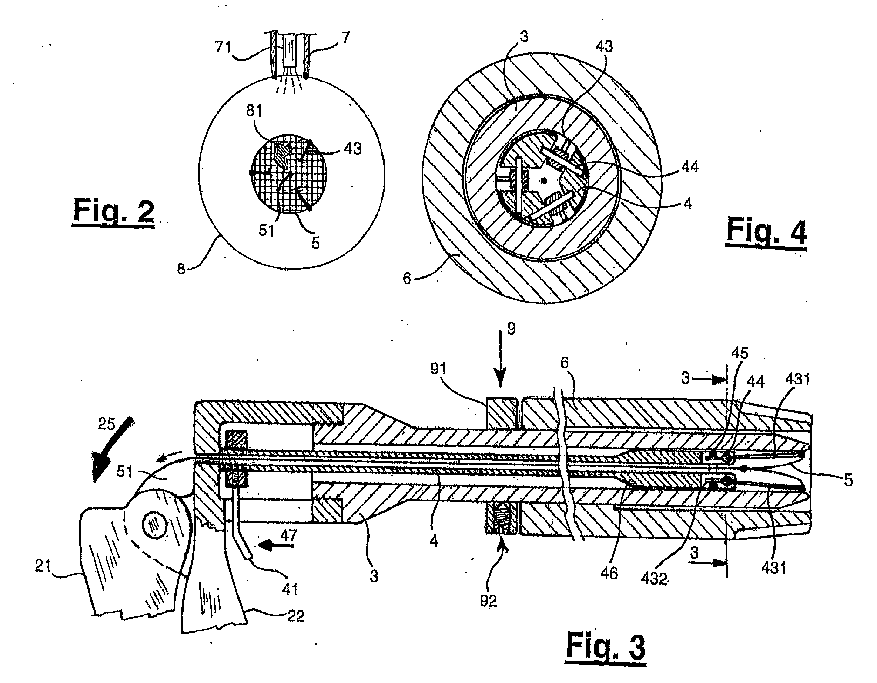

The distal end of the shank 4 is provided with a head-piece 46 which has arms 43 supporting the screen 5. The support arms 43 are for example three in number and are angularly spaced from each other by the same amount. Each arm 43 is pivotably mounted on a pivot 44 which defines a proximal section 432 and a distal section 431. As schematically shown in Figure 3, the proximal sections 432 are pressed elastically towards the axis of the device. For example, the proximal sections 432 are pressed by means of an elastic ring 45 or the like. In any case, when the screen 5 is not unfolded (still with reference to Figure 3), the distal ends of the distal sections 431 of the arms 43 are unable to open up beyond the internal section of the barrel 3.

Preferably, the screen 5 has a substantially circular shape and, as mentioned above, is fastened to the distal end of the arms 43. According to the present invention, the screen 5 is also fastened to a recall member 51. The recall member 51 may be a wire fixed in the centre of the screen. An opposite end of the wire 51 is fixed to a movable part 21 of the handle 2. Conveniently, the wire 51 slides inside an axial through-hole of the shank 4.

The handle 2 is conveniently of the "scissors type" with two half-handles 21, 22 pivotably mounted at 23. In a first position (for example where the two parts 21, 22 of the handle are close together), the wire 51 is in the recall position. In a second position (where, for example, the two parts of the handle 21, 22 are separate from each other) the wire 51 is in the extended position, i.e. allows unfolding of the screen 5.

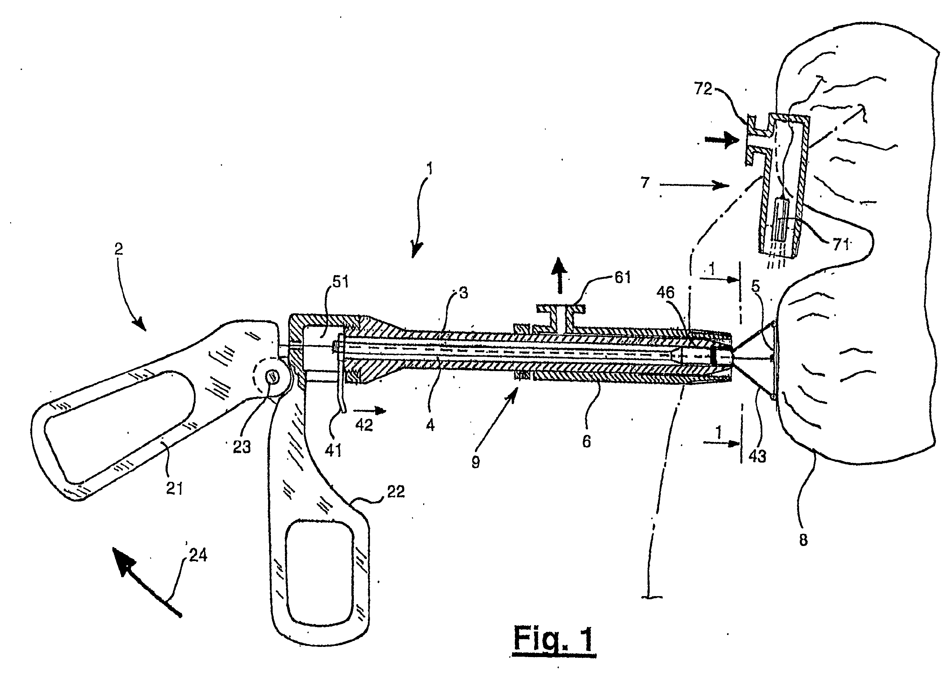

The shank 4 is also movable between a first position (Figure 3) where the support arms 43 are closed inside the barrel 3 and a second position where the support arms are open and unfolded towards the object to be mapped (Figure 1). The shank 4 is displaced axially for example by means of a lever 41 of the trigger type into the vicinity of the handle 2. This allows the surgical instrument of the invention to be operated with one hand.

The barrel 3 has a stop 9 for defining an end-of-travel point so that it is not possible to introduce it mistakenly inside the guide 6 by more than a certain amount. The stop 9 is for example formed by a circular ring 91 and a grub screw 92. The fact that it is possible to modify the position of the stop 9 means that the instrument 1 may be adapted to various types of arthroscopic guides 6.

The operating principle of the device is as follows. An optical cannula with an illumination and image acquisition device 71 and an inlet 72 for entry of a washing fluid (not shown) into the surgical site is introduced. The illumination device 71 illuminates and acquires images of the object to be mapped, for example a cartilaginous lesion 81 of a femoral condyle (or, generally, of an articulation) 8. A arthroscopic surgical guide 6, with an outlet 61 for drawing off the contaminated washing fluid containing impurities, is installed for use with the surgical instrument.

The barrel 3 of the instrument 1 is introduced into the guide 6, keeping the handle 2 in its first position; the shank 4 is also in its first position, as shown in Figures 3 and 4. Once the barrel 3 is at the end of its travel path, the shank 4 is displaced distally (arrow 42 in Figure 1) and the two parts 21, 22 of the handle 20 are rotated in the direction of the arrow 24 so as to separate them. These two movements allow the head-piece 46 of the shank 4 to project out towards the object 81 to be evaluated geometrically. In fact, the distal ends of the distal sections 431 of the arms 43 will be free to open towards the outside of the barrel 3 owing to the elastic force exerted by the elastic ring 45 which presses the proximal sections 432 of the arms 43. In this way the circular screen 5 is unfolded, as shown in Figure 2. By means of the telecamera and the illumination system 71, images of the lesion 81 similar to those of Figure 2 will be acquired and it is now possible to evaluate the form and the extent of the lesion.

Before extracting the surgical instrument 1 from the guide 6 it is appropriate, although not indispensable, to fold up and retract the screen 5 inside the barrel 3 so as not to damage the instrument or the tissues. In order to retract the screen 5 in the form of an overturned umbrella, the handle 21 is rotated in the direction of the arrow 25 of Figure 3 and the shank 4 is displaced proximally in the direction of the arrow 47 by means of the lever 41. The movement of the handle 21 recalls, by means of the wire 51 connected to it, the centre of the screen 5 and axially recalls the arms 43 so that they are able to pass easily into the barrel 3. After these movements of the handle and the shank, the instrument 1 is in the position shown in Figure 3 and may be easily extracted from the arthroscopic guide 6.

Conveniently the instrument 1 is at least partially made of a material which can be easily sterilized, such as steel, aluminium or polymer materials. In the case where it is made of polymer materials it is of the disposable type.

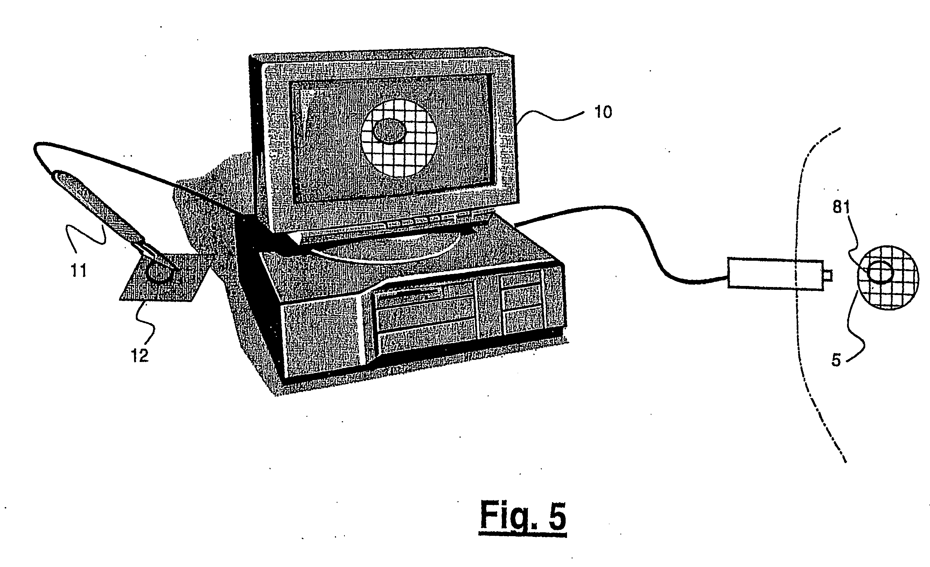

As also shown in Fig. 5, the instrument according to the invention allows mapping of the lesion 81 by arranging on top of the surface affected by the lesion a geometrical sampling system which, in the specific example shown, is a screen 5. This sampling system is clearly visible on the arthroscopic monitor 10 and therefore allows the surgeon, during the operation, to record and archive for subsequent evaluation the digital image which shows the sampling system placed on top of the lesion, and trace, also manually, a precise map of the lesion. In this connection there exist established techniques for evaluating the form and size of the mapped area, so-called morphometric techniques, which may be implemented after the arthroscopic investigation and which are not described in detail here. Conveniently, a computer connected to the arthroscopic monitor operates a cutting instrument 11, for example a laser cutting instrument, for suitably shaping a tissue 12, prepared in a laboratory from primary or stem cells.

The present invention is conveniently used in arthroscopy of the knee, but could also be applied to other areas of the mini-invasive surgical sector which use both arthroscopy and laparoscopy. For example it may also be applied to the evaluation of lesions affecting tissues other than cartilage, for example ulcers or focal lesions of tissues or internal organs which may be observed during laparoscopy operations.

## Claims

1. Surgical instrument (1) for geometrical evaluation of an object (81) inside a body of a human being or animal, the surgical instrument (1) comprising a handle (2), a reference device (5) and means (3, 4, 6, 43, 44) for bringing said reference device (5) into the vicinity of said object (81), said instrument (1) being adapted to co-operate with an image acquisition device for acquiring at least one image of said reference device when it is in the vicinity of said object (81), **characterized in that** said surgical instrument (1) also comprises a recall system (51, 23, 21) for recalling the reference device (5) from an unfolded position into a retracted position.

2. Surgical instrument (1) according to Claim 1, **characterized in that** said reference device (5) is a screen.

3. Surgical instrument (1) according to Claim 2, **characterized in that** the reference device (5) has a dark grey or black colour.

4. Surgical instrument (1) according to any one of the preceding claims, **characterized in that** it comprises a guide barrel (3) and a shank (4) sliding inside said barrel (3), the barrel (3) being able to be inserted inside the body and having a proximal end in the vicinity of the handle (2) and an opposite distal end which is open.

5. Surgical instrument (1) according to Claim 4, **characterized in that** the shank (4) has a head-piece (46) with support arms (43) for supporting said reference device (5).

6. Surgical instrument (1) according to Claim 5, **characterized in that** the support arms (43) are pivotably mounted and are elastically movable between a first position where the reference device (5) is retracted and a second position where the reference device (5) is unfolded in the vicinity of the object (81) to be evaluated.

7. Surgical instrument (1) according to Claim 6, **characterized in that** the shank (4) is adapted to be moved from the position were the reference device (5) is retracted inside the barrel (3) into the position where the reference device (5) is unfolded in the vicinity of the object (81) to be evaluated by means of a trigger (81) situated at the proximal end.

8. Surgical instrument (1) according to Claim 1, **characterized in that** the recall system (51, 23, 21) comprises a wire (51) connected to the reference device (5) and to the handle (2), said wire being slidable inside an axial cavity of the shank (4).

9. Surgical instrument (1) according to any one of the preceding claims, **characterized in that** the handle is preferably of the "scissors type".

10. Surgical instrument (1) according to any one of the preceding claims, **characterized in that** it is at least partially made with polymer material and is of the disposable type.

## Patentansprüche

1. Chirurgisches Instrument (1) zur geometrischen Auswertung eines Objektes (81) innerhalb eines Körpers eines Menschen oder Tieres, wobei das chirurgische Instrument einen Griff (2), eine Referenzvorrichtung (5) und Mittel (3, 4, 6, 43, 44) zum Einbringen der Referenzvorrichtung (5) in die Nähe des Objektes (81) umfasst, wobei das Instrument (1) ausgebildet ist mit einer Bilderfassungseinrichtung zum Erfassen von mindestens einem Bild von der Referenzvorrichtung zusammen zu wirken, wenn sie sich in der Nähe des Objektes (81) befindet,
**dadurch gekennzeichnet,**
**dass** das chirurgische Instrument (1) auch ein Rückholsystem (51, 23, 21) zum Zurückholen der Referenzvorrichtung (5) von einer entfalteten Position in eine eingezogene Position umfasst.

2. Chirurgisches Instrument (1) nach Anspruch 1,
**dadurch gekennzeichnet ,**
**dass** die Referenzvorrichtung (5) eine Bildfläche ist.

3. Chirurgisches Instrument (1) nach Anspruch 2,
**dadurch gekennzeichnet ,**
**dass** die Referenzvorrichtung (5) eine graue oder schwarze Farbe aufweist.

4. Chirurgisches Instrument (1) nach einem der vorhergehenden Ansprüche,
**dadurch gekennzeichnet ,**
**dass** das chirurgische Instrument (1) eine Führungshülse (3) und einen innerhalb der Hülse (3) verschiebbaren Schaft (4) umfasst, wobei die Hülse (3) in den Körper einführbar ist und ein proximales Ende in der Nähe des Griffes (2) und ein gegenüberliegendes distales Ende aufweist, welches offen ist.

5. Chirurgisches Instrument (1) nach Anspruch 4,
**dadurch gekennzeichnet ,**
**dass** der Schaft (4) ein Kopfstück (46) mit Stützarmen (43) zum Stützen der Referenzvorrichtung (5) aufweist.

6. Chirurgisches Instrument (1) nach Anspruch 5,
**dadurch gekennzeichnet ,**
**dass** die Stützarme (43) schwenkbar gelagert sind und elastisch zwischen einer ersten Position, in der die Referenzvorrichtung (5) eingezogen ist, und einer zweiten Position, in der die Referenzvorrichtung (5) in der Nähe des auszuwertenden Objektes (81) entfaltet wird, bewegbar sind.

7. Chirurgisches Instrument (1) nach Anspruch 6,
**dadurch gekennzeichnet ,**
**dass** der Schaft (4) ausgebildet ist von der Position, in der die Referenzvorrichtung innerhalb der Hülse (3) eingezogen ist, in die Position, in der die Referenzvorrichtung (5) in der Nähe des auszuwertenden Objektes (81) entfaltet ist, mittels eines Auslösers (81), der sich am proximalen Ende befindet, bewegt zu werden.

8. Chirurgisches Instrument (1) nach Anspruch 1,
**dadurch gekennzeichnet ,**
**dass** das Rückholsystem (51, 23, 21) einen Draht (51) umfasst, der mit der Referenzvorrichtung (5) und dem Griff (2) verbunden ist, wobei der Draht innerhalb eines axialen Hohlraumes des Schaftes (4) verschiebbar ist.

9. Chirurgisches Instrument (1) nach einem der vorhergehenden Ansprüche,
**dadurch gekennzeichnet ,**
**dass** der Griff vorzugsweise scherenartig ausgebildet ist.

10. Chirurgisches Instrument (1) nach einem der vorhergehenden Ansprüche,
**dadurch gekennzeichnet ,**
**dass** das chirurgische Instrument zumindest teilweise aus einem Polymermaterial besteht und ein Einweg-Produkt ist.

## Revendications

1. Instrument chirurgical (1) pour l'évaluation géométrique d'un objet (81) à l'intérieur d'un corps d'un être humain ou d'un animal, l'instrument chirurgical (1) comprenant une poignée (2), un dispositif de référence (5) et des moyens (3, 4, 6, 43, 44) pour amener ledit dispositif de référence (5) dans le voisinage dudit objet (81), ledit instrument (1) étant conçu pour coopérer avec un dispositif d'acquisition d'image pour acquérir au moins une image dudit dispositif de référence lorsqu'il est dans le voisinage dudit objet (81), **caractérisé en ce que** ledit instrument chirurgical (1) comprend également un système de rappel (51, 23, 21) pour ramener le dispositif de référence (5) d'une position déployée dans une position rétractée.

2. Instrument chirurgical (1) selon la revendication 1, **caractérisé en ce que** ledit dispositif de référence (5) est une grille.

3. Instrument chirurgical (1) selon la revendication 2, **caractérisé en ce que** le dispositif de référence (5) a une couleur gris foncé ou noire.

4. Instrument chirurgical (1) selon l'une quelconque des revendications précédentes, **caractérisé en ce qu'**il comprend un cylindre de guidage (3) et une tige (4) coulissant à l'intérieur dudit cylindre (3), le cylindre (3) étant capable d'être inséré à l'intérieur du corps et ayant une extrémité proximale dans le voisinage de la poignée (2) et une extrémité distale opposée qui est ouverte.

5. Instrument chirurgical (1) selon la revendication 4, **caractérisé en ce que** la tige (4) a une tête (46) avec des bras de support (43) pour supporter ledit dispositif de référence (5).

6. Instrument chirurgical (1) selon la revendication 5, **caractérisé en ce que** les bras de support (43) sont montés de manière pivotante et peuvent être déplacés de manière élastique entre une première position où le dispositif de référence (5) est rétracté et une deuxième position où le dispositif de référence (5) est déployé dans le voisinage de l'objet (81) à évaluer.

7. Instrument chirurgical (1) selon la revendication 6, **caractérisé en ce que** la tige (4) est conçue pour être déplacée de la position où le dispositif de référence (5) est rétracté à l'intérieur du cylindre (3) dans la position où le dispositif de référence (5) est déployé dans le voisinage de l'objet (81) à évaluer au moyen d'une gâchette (81) située à l'extrémité proximale.

8. Instrument chirurgical (1) selon la revendication 1, **caractérisé en ce que** le système de rappel (51, 23, 21) comprend un câble (51) relié au dispositif de référence (5) et à la poignée (2), ledit câble étant capable de coulisser à l'intérieur d'une cavité axiale de la tige (4).

9. Instrument chirurgical (1) selon l'une quelconque des revendications précédentes, **caractérisé en ce que** la poignée est de préférence du type « ciseaux ».

10. Instrument chirurgical (1) selon l'une quelconque des revendications précédentes, **caractérisé en ce qu'**il est au moins partiellement réalisé en un matériau polymère et est du type jetable.
